# EUROPEAN PATENT SPECIFICATION

(11) **EP 1 245 202 B1**
(45) Date of publication and mention of the grant of the patent: **04.08.2004**
(21) Application number: 01610036.4
(22) Date of filing: 27.03.2001
(51) Int. Cl.: A61F 2/06

(54) **An aortic graft device**
Gefässtransplantat für die Aorta
Greffe aortique

(43) Date of publication of application: 02.10.2002
(73) Proprietor: William Cook Europe ApS, 4632 Bjaeverskov (DK); Cook Incorporated, Bloomington, Indiana 47402 (US)
(72) Inventor: Koul, Bansi Lal, 22647 Lund (SE); Ivancev, Krasnodar, 22221 Lund (SE)
(74) Representative: Jorgensen, Bjorn Barker

(56) References cited:
- EP-A- 0 955 019
- WO-A-96/39999
- WO-A-98/33638
- US-A- 6 036 723

## Description

The present invention relates to an aortic graft device having a proximal end and a first connecting means at a distal opening.

An aortic graft is an endovascular prosthesis for placement in aorta in a weakened area, such as an aneurysm or an aortic dissection. An aortic aneurysm is an abnormal dilation or enlargement of the arterial wall of the aorta, and an dissection relates to intimal tear of the aorta and subsequent propagation of blood in the aortic wall distally or proximally over varying length. If a significantly large aneurysm or aortic dissection is not surgically or medically treated, it can rupture with abrupt fatal blood loss either into the abdominal or thoracic cavity.

An aortic stent graft is usually made of a tube of pliable material provided with a stent for anchoring the graft in its intended position within the blood vessel by exerting an outwardly directed radial pressure against the surrounding aortic wall. One of the requirements for successful anchoring of the graft is that the anchoring stent is placed against a relatively healthy aortic wall that can withstand the radial pressure for years.

Patients who need treatment for aortic aneurysms are often elderly with concomitant illnesses which increase the risk of complications associated with aortic aneumysm surgery. Most prior art stent grafts are designed for abdominal aortic aneurysms involving infra- and pararenal abdominal aorta. Examples of such grafts are described in US patents 5,984,955 and 6,016,810. Many of these grafts are bifucated and extended to the iliac arteries. Aortic grafts of these types can typically be placed using transluminal, minimally invasive procedures where the grafts are percutaneously introduced via a femoral puncture site, and the entire procedure can be performed using local anesthesia.

With respect to aortic grafts for the repair of thoracoabdominal aortic aneurysms (aneurysms in the descending thoracic aorta) and other thoracic aortic aneurysms (aneurysms in the ascending thoracic aorta including the aortic arch) only a few proposals for minimally invasive procedures have been made so far.

US patent 6,099,548 proposes to advance the graft into the ascending thoracic aorta and to lock it to the aortic wall by expanding a stent in the proximal end of the graft. However, adjacent the heart the flow rate of blood through aorta is so high that the risk of dislocation of the proximal stent is considerable.

For aneurysms of the aorta involving the ascending aorta, the aortic arch and the descending thoracic aorta a staged surgical procedure is usually employed. In a first stage (stage I) the ascending aorta and the arch of the aorta are replaced using a vascular graft and a non-stented vascular prosthesis is left hanging in the aneurysmatic proximally descending thoracic aorta, also called the elephant trunk technique. At a second stage, (stage II) the elephant trunk is extended by another vascular graft which is anastomosed distally to thoracic or abdominal aorta depending upon the location of the aneurysms. The first stage of the operation is almost always done under cardiopulmonary bypass, deep hypothermia and total circulatory arrest. During the period of total circulatory arrest the arch of the aorta is changed and the great vessels of the neck namely the brachiocephalic artery, the left communic artery and the subclavian artery are anastomosed to the vascular prosthesis. A limitation of this surgery is the time period for which the brain and the spinal cord can be kept anoxic. Periods up to 45 minutes at 16°C are well tolerated by the brain although these margins of safety can be increased slightly by employing retrograde perfusion technique during the procedure.

The stage II procedure, involving extension of the elephant trunk technique to the descending thoracic aorta, is accomplished through a long posterolateral thoracotomy with or without cardiopulmonary bypass.

Dissections of type A where the ascending aorta is involved in the dissection pose a particular problem. Type A acute aortic dissection which usually starts with an intimal tear in the ascending aorta is regarded as a surgical emergency. If untreated 75 % of these patients die within 24 hours and almost 90 % are dead within one week.

A purpose of the present invention is to improve the thoracic aortic aneurysm surgery, especially when acute aortic dissection is involved.

With a view to this the aortic graft device according to the invention is characterized in that it has an anchoring area positioned between a proximal portion extending to said proximal end and a distal portion extending to said distal opening, that the proximal portion is corrugated, and that the distal portion is at least partially non-corrugated.

The corrugations on the proximal portion of the graft facilitates setting of the proximal portion in a curvature following the path of the ascending aorta in the particular patient. This saves some time. After anchoring the graft device to the aortic wall the proximal portion of the graft can be located in the ascending aorta and the distal portion of the graft device part can be left hanging loose in the descending aorta. Time consuming suturing of a secondary graft to the graft device part is avoided.

The non-corrugated area on the distal portion makes it possible to connect the distal portion to a distal extention graft or to the aortic wall at a subsequent operation. When the surgeon has performed the anchoring of the aortic graft device he can decide to insert a distal graft part right away if time allows this, or to postpone placement of a secondary graft part or an alternative connection of the distal end of the graft device part to the aortic wall to another operation. The surgeon also fixes the proximal end of the graft device part to the desired site of the aortic wall in the ascending aorta.

The anchoring area positioned between the ascending and the distal portions of the graft device part make it possible for the graft to be fixed to a portion of the aortic wall in vicinity of the three branch arteries on the aortic arch. The anchoring can be made to the distal side of the arteria subclavia at least at the upper portion of the aortic arch and possibly in an annular pattern following the aortic wall.

The non-corrugated distal portion of the aortic graft device can be extended into the descending thoracic aorta in a second stage or in the same setting when the ascending aorta and aortic arch are being replaced. The distal portion of the aortic graft device may in itself be subsequently long to exclude the descending thoracic aortic aneurysm by itself.

With the suitable surgical intervention the hospital mortality can be reduced down to about 20 % or lower.

The distal portion of the aortic graft device may be non-corrugated partially or wholly along its full length. In an embodiment the distal portion of the aortic graft device is non-corrugated at least at said first connecting means, and preferably along its full length from said anchoring area to said distal opening. The non-corrugated area makes it easier to obtain a blood tight connection between the aortic graft device and the tubular member connected to it.

The distal portion of the aortic graft device is provided with at least one stent, and preferably provided with at least two stents, which are located near the anchoring area and at the distal opening, respectively. The stent resiliently acts on the distal portion to keep it open and non-kinking.

In a further embodiment the distal portion of the graft device has a distal end area which is uncorrugated and unstented and has a length of at least 20 mm. The distal end area can be utilized to couple or anastomose the graft device to either a distal graft or to the aortic wall in the descending thoracic aorta. This part of the aortic graft device can likewise be used for endovascular extention of the aortic graft device into the descending thoracic aorta. It is possible to perform suturing in the distal end area, but it is likewise possible to use the smooth surface character in the distal end area to obtain a pressure-thight sealing between the graft device and the distal graft. At a stage II step as mentioned above there is only a low or no risk of the distal portion getting inverted into the aortic arch.

The distal portion of the aortic graft device is preferably supported by stent at least along the majority of its length. By the stenting the distal portion is kept fully open, also when it is subjected to compressing or kinking actions on the exterior of the distal portion which is hanging freely floating in the aortic vessel downstream of the anchoring area. This is in particular useful when a distal graft is to be connected to the aortic graft device at separate surgery.

In order to facilitate handling and placement of the graft device at least the proximal portion of the aortic graft device is precurved. The precurved shape reduces the size of bending forces required to keep the proximal portion in position during suturing of the anchoring area and thus minimizes the time spent on correcting the location of the aortic graft device during suturing.

In a preferred embodiment a wedge-shaped area of graft side wall in the proximal portion has been removed and the exposed opposite rim areas have been joined to each other. The removal of the wedge-shaped area results in a distinct or sharp bend in the graft part at the place of the removed material and allows on the one hand manufacturing of the graft part as a straight tubular part with uniform properties and on the other hand the more sharp bend can be obtained in a comparatively easy manner. This facilitates exact repair of the ascending aorta and the aorta valve. Moreover, the embodiment would also prevent kinking of the graft device.

It is preferred that the proximal unstented portion has a length in the range of from 10 to 15 cm, that said distal portion has a stented length in the range of from 5 to 10 cm including said distal end area having a length in the range of from 2 to 3 cm. These features make the aortic graft device flexible and well suited to accept different kinds of fixation modes in the descending thoracic aorta either directly to the descending thoracic aorta or for extension with another vascular prosthesis or by endovascular extension with the help of a stented graft. In patients with type A aortic dissection or type B aortic dessection with or without antegrade extension of the dissection into the arch and ascending aorta the device may be used with and without the cardiopulmonary bypass to seal the primary or secondary entry sites at different levels. This would promote better hemostasis during surgery and prevent further progress of dissection and/or treat dynamic obstruction in the descending thoracic aorta. Like in the descending thoracic aortic aneurysms the aortic graft device can subsequently be extended endovascularly or by open surgical technique to exclude aneurysms distal to the aortic graft device in the thoracic and abdominal aorta.

In order to improve blocking of dissections at a particular location in the aorta the anchoring area can comprise an annular area encircling the aortic graft device. This allows for a fixation of the graft to the aortic wall along a full cirumference of the wall.

It is preferred that said annular area is uncorrugated and unstented and has a length of at least 10 mm. The surgeon can quickly fix this annular anchoring area to the aortic wall because the area is free from pre-manufactured corrugations and stents and has a length that provides easy access to a contact point between graft and wall when the suture is to be applied.

The anchoring area can in an embodiment include a lateral area which extends away from the annular area in direction of the proximal portion. This lateral area can be positioned next to the three branch arteries and quickly be fixed to the aortic wall.

In a further embodiment an aortic arc portion, such as said lateral area, of the aortic graft device has a marking, such as radiopaque markers or a surface coloring distinct from the remainder of the aortic graft device. The markers prevent accidental placement of the concave portion of the aortic graft device towards the aortic arch.

In yet a further development the lateral area includes one or several openings that can be connected to the aortic arch branches by direct anastomosis or by use of a stabling device.

The aortic graft device can be supplied in or be set in a mounting state where the proximal portion is everted into said distal portion to present said annular anchoring area as a rim area ready for suturing to the proximal descending thoracic aorta distal to the left subclavian artery. This may be done within total circulatory arrest.

A dissection of type A can extend into the area of the heart valve and depending on the actual circumstances, such as whether a coronary artery is involved in the dissection, it can be desirable to repair the heart valve, and in this case the graft device preferably also includes a proximal graft extension with a heart valve.

When the graft device includes a heart valve it is preferred that the aortic graft device has a proximal opening, and that the end portion with the heart valve is a separate part to be fixed to said aortic graft device at said proximal opening. This division of the graft device allows the surgeon to split the surgery into separate rounds where the time consuming fixation of the proximal graft extension to the heart and coronary arteries can be performed during cardiopulmonary bypass without circulatory arrest. Then, in a second round, the aortic graft device can be applied after establishing systemic circulatory arrest, but the duration of circulatory arrest is considerably shortened by performing a large part of the surgery required during said first round.

As mentioned above the distal portion of the aortic graft device can be connected to the aortic wall in different manners. In one embodiment the graft device includes a distal graft extension with a second connecting means that by engagement with said first connecting means connects said distal graft extension to said aortic graft device. Use of the distal graft extension can often be a preferred alternative to suturing of the distal portion to the aortic wall. The second connecting means makes possible a quick establishment of the interconnection between the parts.

This embodiment can be further developed so that the engagement involve geometrically interlocking parts on said first and second connecting means, respectively, preferably so that the engagement is enhanced when said distal graft extension is pulled in the distal direction. The geometrical locking of the two parts can provide better long term durability of the graft device which continously is subjected to the pressure pulses produced by the heart beats.

Embodiments of the present invention are explained in more detail in the following with reference to the highly schematical drawings, on which
Fig. 1 and 2 depict two embodiments of an aortic graft device according to the present invention,
Figs. 3-8 illustrate an example of deployment of the device in Fig. 1, and
Fig. 9 illustrate another embodiment of the device according to the present invention.

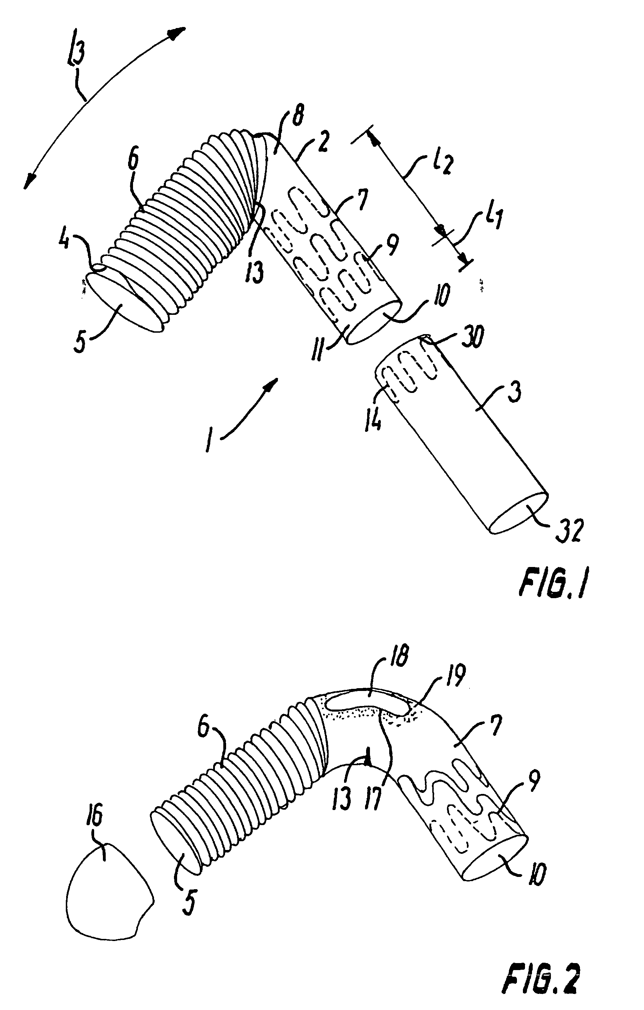

An aortic graft device illustrated in Fig. 1 is generally designated 1 and includes an aortic graft device 2 and an optional distal graft extension 3 for possible subsequent use to exclude a remaining descending thoracic aortic aneurysm or dissection. The aortic graft device 2 is tubular and has a proximal end 4 with a proximal opening 5. In the present context proximal is used for something located closer to the heart or to the side of the heart whereas distal is used for something located more distant from the heart.

The graft device and possible graft extensions are tubular and can be made of a pliable material, such as expanded polytetrafluoroethylene (PTFE), woven polyester or another biocompatible material for long term stability in the vascular system. Graft materials are well known in the art and the material can also include biodegradeable strands as part of the material.

The aortic graft device has an proximal portion 6 and a distal portion 7. An anchoring area 8 is positioned in between the two portions 6, 7. The proximal portion 6 is at least partially corrugated (crimped) or folded in order to promote setting of the proximal portion in a curved shape and flexibility of the graft in use.

The distal portion 7 is at least partially non-corrugated. It can have corrugations along part of its length and/or circumference, but preferably it is non-corrugated along its complete surface. The distal portion 7 can be unstented along its entire length, but preferrably it is at least partially stented by a stent 9 located at a distal opening 10. Stent 9 is of well known construction. It can e.g. be a so-called Gianturco Z-stent or another stent of expandable or self-expandable type which typically is of nitinol, stainless steel or another biocompatible material. Stent 9 is typically placed inside the tubular graft material, but can alternatively be placed on the outside of the tubular material which is then fastened to the stent struts, or the stent can be integrated in the graft material itself.

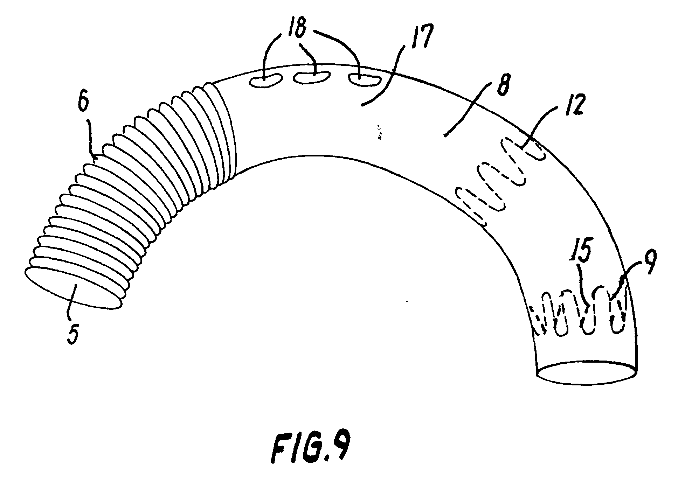

Next to distal opening 10 the distal portion has a distal end area 11 which is unstented and can have a length 11 in the range from 3 mm to 40 mm, preferably from 20 mm to 30 mm. Stent 9 can extend continuosly from distal end area 11 to the anchoring area 8. As depicted in Fig. 9 there can also be used two (or more) separate stents 9, 12 located at a distance from each other. The stent or stents keep the anchoring area in a stretched tubular shape ready for suturing and keep the distal opening in an open state that is accessible from the distal part of the aorta.

Fig. 1 also illustrates that the distal end of the graft is unstented which would facilitate its anchoring to the extension grafts which may be both stented or unstented. The stented distal portion maintains the geometry of this graft and permits easy endovascular extension of the device into the descending thoracic aorta without risk for evertion into the aortic arch. The stented distal portion also provides radiographic visualization during extension procedures thereby reducing the amount of contrast material injected and exact approximation of the extension grafts into the aortic graft device. The stents in the distal portion also permits exact follow-up of these patients over years without subjecting these patients to CT scans of the chest with contrast since the overlapping of the stent grafts in the descending thoracic aorta can be monitored by simple X-ray of the chest in the posterior-anterior view and the lateral view. The stents graft in the distal portion of the aortic graft also provide safety where the graft material does not increase in width toward time thereby putting the extension grafts at risk of displacement and migration and thereby failure to exclude aneurysm and subsequent risk of rupture and death.

In Fig. 1 the stented length 12 of the distal portion is in the range of 5 cm to 10 cm. The proximal portion has a length 13 in the range from 11 cm to 17 cm. The diameter of the graft device is in the range from 22 mm to 38 mm, preferably from 30 mm to 34 mm, such as about 32 mm. The tubular graft material has typically an even diameter along its length when it is manufactured, and some variations in diameter can result from the crimping of the proximal portion. However, it is also possible to make the graft of two separate tubular parts of different diameter if the proximal portion is to be of a diameter different from the distal portion of the graft device.

At the lower (concave) side of the tubular graft a wedge-shaped area of the material has been cut away and the rim areas 13 have been joined, such as by suturing. This has been made to adapt the aortic graft device to the shape of the aortic arch, thus preventing kinking of the graft and promoting exact designing of the width and configuration of the graft to the ascending aorta and its the sinotubular junction.

The distal extension graft 3 has a connecting means 14 depicted as a stent in its proximal end. When the distal extension graft has been inserted through distal opening 10 into distal portion 7 and has been expanded, whether by self-expansion or balloon expansion, the connecting means 14 engages with stent 9 acting as a first connecting means on the aortic graft device. The engagement can be of the frictional type produced by the radially outward pressure from stent 14 on the inside of the distal portion, but it is preferred that the mutual engagement involves geometrically interlocking parts, such as hooks 15 on stent 9 (Fig. 9). The hooks extend obliquely in the proximal direction. The hooks penetrate the graft material of graft 3 and engage with the struts of stent 14 when the graft 3 is pulled slightly in the distal direction.

In the following description of embodiments the same reference numerals are used for details of the same type.

In Fig. 2 the graft device also includes an end portion 16 with a heart valve. The stent 9 runs in a spiral pattern from one end to the other of the distal portion 7. The anchoring area includes a lateral area 17 extending annularly around a lateral opening 18 to be positioned at the three major branch arteries from the aortic arch. Lateral opening 18 can be part of the graft device as manufactured or it can be cut by the surgeon after he has opened the patient and ascertined the actual dimensions and locations of the proximal end opening of the branch arteries. In the latter case the lateral area can have a marking 19, such as a coloring, clearly indicating where the upwards facing lateral anchoring area is located on the graft device. This allows the surgeon to immediately position the graft device correctly and also helps to ensure that the cutting of the lateral opening or openings is only done in the unstented anchoring area. Fig. 9 illustrates an embodiment with three lateral openings 18.

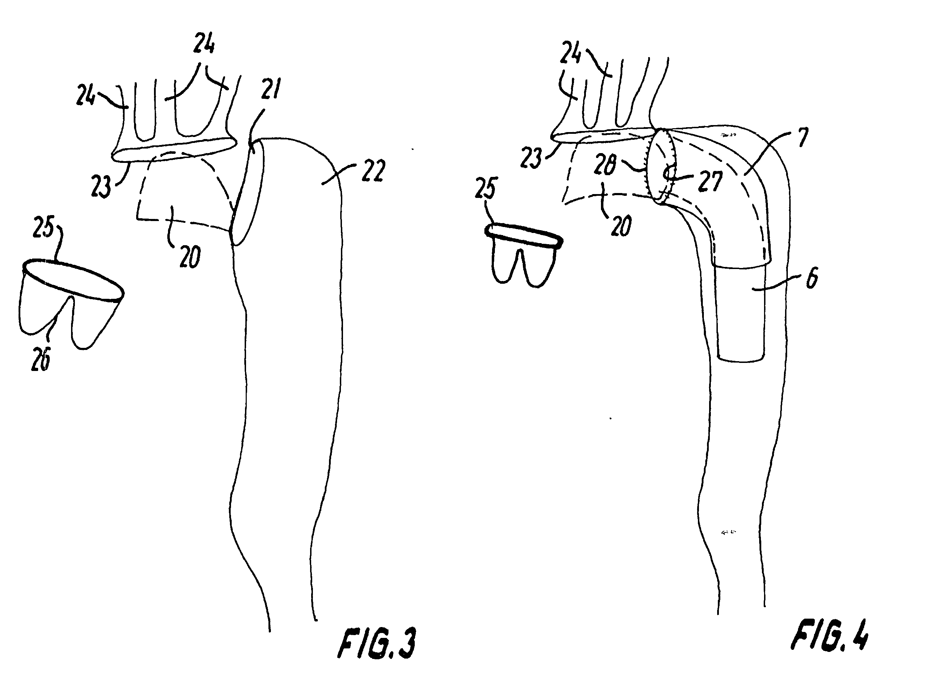

With reference to Figs. 3-8 there is in the following shortly described an example of how the aortic graft device can be deployed.

After cooling the patient down and exposing the aortic arc through a sternotomy and performing cardiopulmonary bypass or systemic circulatory arrest the proximal portion of aorta is resected, thereby exposing an proximal end opening 21 on the descending aorta 22 and a proximal end area 23 at the three major branch arteries 24 and a distal end opening 25 at the heart valve 26.

The graft device is in a mounting state with the proximal portion 6 inverted down into the descending protion 7 (Fig. 4). The graft device is inserted into the descending aorta and is placed with a rim area 27 at the end opening 21 and then the rim area 27 on the graft is fixed to the aortic wall, such as by suturing 28 or stabling.

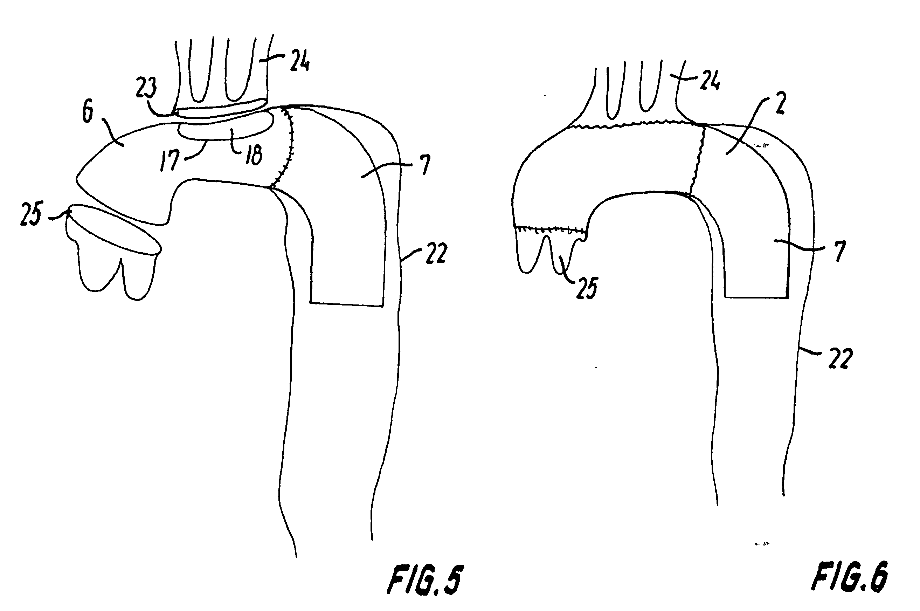

After fixing of the graft to the aottic wall the proximal portion of the graft is inverted to the position illustrated in Fig. 5 with lateral opening 18 located at the proximal end area 23. Then this end area 23 is joined with the lateral anchoring area 17, such as by suturing or stabling. At this point in time the surgeon can decide to insert the secondary graft part through the proximal opening 5 and mount it in the ascending aorta and/or to the distal portion of the aortic graft device. In many cases the decision will be to postpose the placement of the secondary graft part to a separate surgery in order to reestablish circulation as quickly as possible. Then the rim area at the proximal opening 5 is joined with the rim area at the distal end opening 25, such as by suturing or stabling.

The graft device is then in the state illustrated in Fig. 6 where the circulation can be reestablished. The distal portion is hanging loose into the aorta. Depending on the condition and desires of the patient it is then possible to proceed in alternative ways. One possibility is to open the patient from the side and secure the end area 11 to the aortic wall, such as by suturing or stabling or stenting. Another possibility is to add the distal graft extension 3 which can be done either by percutaneously introducing (e.g. femorally) and intraluminally advancing graft extension 3 with a minimally invasive procedure or by performing open surgery.

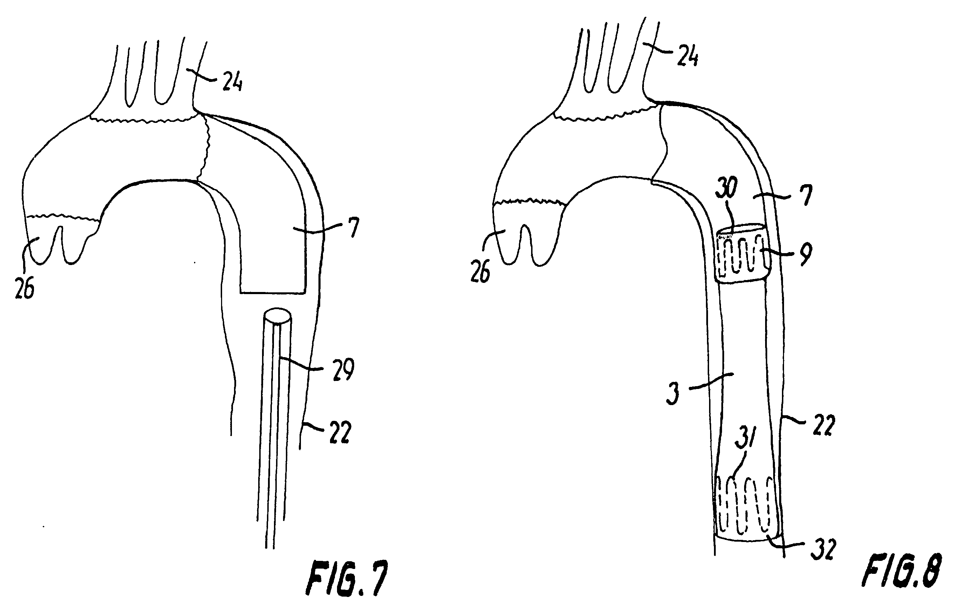

Fig. 7 illustrates the introduction of the distal graft extension 3 held in a radially reduced state in an introducer 29. When the proximal end 30 of graft extension 3 is correctly located in the distal portion 7 the proximal end 30 is released from or expanded by the introducer so that the first and second connecting means are brought into secure mutual engagement. Then a distal end 32 of the distal graft extension 3 is located at the desired site of the aorta and is fixed thereto, either by suturing or stabling or by stenting. Fig. 7 illustrates stenting with a distalmost stent 31 in the distal end.

An example of the procedure followed during placement of the aortic graft device is shortly described in the following.

The patient is subjected to midline sternotomy and conventional cardiopulmonary bypass. Then the patient is cooled to about 16°C and the patient's aorta is cross-clamped proximal to the origin of the brachiocephalic artery. During the period of cooling the ascending aorta with or without aortic valve is reconstructed or repaired using various techniques namely Bentall procedure, aortic root remodelling, sinotubular junction remodelling etc. At 16°C- 18°C body temperature with or without retrograde cerebral perfusion the cardiopulmonary bypass is stopped and the patient is exsanguinated. The arch of the aorta is excised and the proximal end of the descending thoracic aorta exposed. The corrugated portion of the aortic graft device is inverted into the stented portion and the composite graft lowered in the proximal descending thoracic aorta. The unstented portion of the aortic graft device is sutured to the proximal descending thoracic aorta and the corrugated portion of the graft everted. Under continued total circulatory arrest one or multiple holes are made under the convexe portion of the unstented corrugated portion fur anastomoses to the branches of the aortic arch. This can be performed by sewing aortic branches as one common foot or in usually using either suture or stapling technique. The aortic graft device proximal to the origin of the brachiocephalic artery is cross-clamped after deairing and cardiopulmonary bypass reinitiated. The patient is warmed to 37°C and the aortic graft device sutured or stapled to the ascending aortic vascular graft.

The aortic graft device may e.g. be used for treatment of ascending aortic aneurysms, aortic arch aneurysms and descending thoracic aortic aneurysms and/or type A and B acute aortic dissections or subacute and chronic type A or B aortic dissections in single or multiple stages.

The aortic graft device can be pre-manufactured with one or several openings at the presumptive aortic arch anastomose stick side for direct suture or stapling of the aortic graft device to the aortic arch vessels, and the aortic graft device can be anchored to the ascending aortic graft or ascending aorta and the descending aortic graft or descending aorta by direct anastomosis or by stapling or by endovascular techniques.

The aortic graft device can be provided as such or in a mounted state for delivery into the descending thoracic aorta while the patient is still on cardiopulmonary bypass thereby keeping the total circulatory arrest time to minimum.

The aortic graft device can be extended proximally or distally by extension grafts which are mounted with stents and these stents are supplemented with hooks and barbs for better fixation and tethering to the aortic graft device. The distal or proximal extension graft may have a connection mechanism as stents in the end facing the aortic graft device. These stents may be modified with hooks and/or barbs projecting outside the prosthesis and increasing the tethering of the extension grafts into the distal or proximal part of the aortic graft device. In a similar way the aortic graft device may be modified so that the stents in the distal portion of the aortic graft device are provided with hooks and barbs directed proximally and intraluminally. These hooks and barbs of the aortic graft device and the extension graft interdigitae and provide better fixation between the two grafts without need for supplementary endovascular procedures.

Details of the above mentioned embodiments can be combined into other embodiments within the scope of the present invention as defined in the claims.

## Claims

1. An aortic graft device having a proximal end and a first connecting means at a distal opening, **characterized in that** the aortic graft device has an anchoring area positioned between a proximal portion extending to said proximal end and a distal portion extending to said distal opening, that the proximal portion is corrugated, and that the distal portion is at least partially non-corrugated.

2. An aortic graft device according to claim 1, **characterized in that** the distal portion of the aortic graft device is non-corrugated at least at said first connecting means, and preferably along its full length from said anchoring area to said distal opening.

3. An aortic graft device according to claim 1 or 2, **characterized in that** the distal portion of the aortic graft device is provided with at least one stent, and preferably provided with at least two stents, which are located near the anchoring area and at the distal opening, respectively.

4. An aortic graft device according to claim 3, **characterized in that** the distal portion of the graft device has a distal end area which is uncorrugated and unstented and has a length of at least 20 mm.

5. An aortic graft device according to any one of claims 1 to 4, **characterized in that** the distal portion of the aortic graft device is supported by stent at least along the majority of its length.

6. An aortic graft device according to any one of claims 1 to 5, **characterized in that** at least the proximal portion of the aortic graft device is precurved.

7. An aortic graft device according to any one of claims 1 to 6, **characterized in that** in the proximal portion a wedge-shaped area of graft side wall has been removed and the exposed opposite rim areas joined to each other.

8. An aortic graft device according to any one of claims 4 to 7, **characterized in that** said proximal portion has a length in the range from 10 to 15 cm, and that said distal portion has a length in the range from 5 to 10 cm including said distal end area having a length in the range from 2 to 3 cm.

9. An aortic graft device according to any one of claims 1 to 8, **characterized in that** said anchoring area comprises an annular area encircling the aortic graft device.

10. An aortic graft device according to any one of claims 1 to 9, **characterized in that** said annular area is uncorrugated and unstented and has a length of at least 10 mm.

11. An aortic graft device according to claim 9 or 10, **characterized in that** said anchoring area includes a lateral area extending away from the annular area in direction of the proximal portion.

12. An aortic graft device according to claim 11, **characterized in that** an aortic arc portion, such as said lateral area, of the aortic graft device has a marking, such as radiopaque markers or a surface coloring distinct from the remainder of the aortic graft device.

13. An aortic graft device according to claim 11 or 12, **characterized in that** said lateral area includes a lateral opening.

14. An aortic graft device according to any one of claims 1-13, **characterized in that** in a mounting state of said aortic graft device the proximal portion has been everted into said distal portion to present said annular anchoring area as a rim area.

15. An aortic graft device according to any one of claims 1 to 14, **characterized in that** the graft device includes a proximal extension with a heart valve.

16. An aortic graft device according to claim 15, **characterized in that** the aortic graft device has a proximal opening, and that the proximal extension with the heart valve is a separate part to be fixed to said aortic graft device at said proximal opening.

17. An aortic graft device according to any one of the preceding claims, **characterized in that** the graft device includes a distal graft extension with a second connecting means that by engagement with said first connecting means connects said distal graft extension to said aortic graft device.

18. An aortic graft device according to claim 17, **characterized in that** the engagement involves geometrically interlocking parts on said first and second connecting means, respectively, preferably so that the engagement is enhanced when said distal graft extension is pulled in the distal direction.

## Revendications

1. Dispositif de greffe aortique ayant une extrémité proximale et un premier moyen de raccordement au niveau d'une ouverture distale, **caractérisé en ce que** le dispositif de greffe aortique a une zone d'ancrage placée entre une portion proximale s'étendant vers ladite extrémité proximale et une portion distale s'étendant vers ladite ouverture distale, **en ce que** la portion proximale est ondulée et **en ce que** la portion distale est au moins partiellement non ondulée.

2. Dispositif de greffe aortique selon la revendication 1, **caractérisé en ce que** la portion distale du dispositif de greffe aortique n'est pas ondulée au moins au niveau dudit premier moyen de raccordement, et de préférence le long de toute sa longueur depuis ladite zone d'ancrage à ladite ouverture distale.

3. Dispositif de greffe aortique selon la revendication 1 ou 2, **caractérisé en ce que** la portion distale du dispositif de greffe aortique est munie d'au moins une endoprothèse vasculaire, et de préférence munie d'au moins deux endoprothèses vasculaires, qui sont situées à proximité de la zone d'ancrage et au niveau d'une ouverture distale, respectivement.

4. Dispositif de greffe aortique selon la revendication 3, **caractérisé en ce que** la portion distale du dispositif de greffe a une zone d'extrémité distale qui n'est pas ondulée et pas munie d'endoprothèse vasculaire et qui a une longueur d'au moins 20 mm.

5. Dispositif de greffe aortique selon l'une quelconque des revendications 1 à 4, **caractérisé en ce que** la portion distale du dispositif de greffe aortique est soutenu par l'endoprothèse vasculaire au moins le long de la majorité de sa longueur.

6. Dispositif de greffe aortique selon l'une quelconque des revendications 1 à 5, **caractérisé en ce que** au moins la portion proximale du dispositif de greffe aortique est préalablement courbé.

7. Dispositif de greffe aortique selon l'une quelconque des revendications 1 à 6, caractérisé en ce dans la portion proximale une zone cunéiforme de la paroi latérale de la greffe a été enlevée et les zones de rebord opposé exposées ont été liées les unes aux autres.

8. Dispositif de greffe aortique selon l'une quelconque des revendications 4 à 7, **caractérisé en ce que** ladite portion proximale a une longueur dans la plage allant de 10 à 15 cm, et **en ce que** ladite portion distale a une longueur dans la plage allant de 5 à 10 cm comprenant ladite zone d'extrémité distale ayant une longueur dans la plage allant de 2 à 3 cm.

9. Dispositif de greffe aortique selon l'une quelconque des revendications 1 à 8, **caractérisé en ce que** ladite zone d'ancrage comprend une zone annulaire encerclant le dispositif de greffe aortique.

10. Dispositif de greffe aortique selon l'une quelconque des revendications 1 à 9, **caractérisé en ce que** ladite zone annulaire n'est pas ondulée et ne comprend pas d'endoprothèse vasculaire et a une longueur d'au moins 10 mm.

11. Dispositif de greffe aortique selon la revendication 9 ou 10, **caractérisé en ce que** ladite zone d'ancrage comprend une zone latérale s'éloignant de la zone annulaire en direction de la portion proximale.

12. Dispositif de greffe aortique selon la revendication 11, **caractérisé en ce que** la portion d'arc aortique, telle que ladite zone latérale, du dispositif de greffe aortique a une marque, telle que des marqueurs radio-opaques ou une coloration de surface distincte du reste du dispositif de greffe aortique.

13. Dispositif de greffe aortique selon la revendication 11 ou 12, **caractérisé en ce que** ladite zone latérale comprend une ouverture latérale.

14. Dispositif de greffe aortique selon l'une quelconque des revendications 1 à 13, **caractérisé en ce que** dans un état de montage dudit dispositif de greffe aortique, la portion proximale a été renversée dans ladite portion distale pour présenter ladite zone d'ancrage annulaire comme une zone de rebord.

15. Dispositif de greffe aortique selon l'une quelconque des revendications 1 à 14, **caractérisé en ce que** le dispositif de greffe comprend une extension proximale avec une valvule cardiaque.

16. Dispositif de greffe aortique selon la revendication 15, **caractérisé en ce que** le dispositif de greffe aortique a une ouverture proximale, et **en ce que** l'extension proximale avec la valvule cardiaque est une partie séparée destinée à être fixée audit dispositif de greffe aortique au niveau de ladite ouverture proximale.

17. Dispositif de greffe aortique selon l'une quelconque des revendications précédentes, **caractérisé en ce que** le dispositif de greffe comprend une extension de greffe distale avec un second moyen de raccordement qui, par engagement avec ledit premier moyen de raccordement, relie ladite extension de greffe distale audit dispositif de greffe aortique.

18. Dispositif de greffe aortique selon la revendication 17, **caractérisé en ce que** l'engagement implique des parties de verrouillage réciproque géométriquement sur lesdits premier et second moyens de raccordement, respectivement, de préférence de sorte que l'engagement est amélioré lorsque ladite extension de greffe distale est tirée dans la direction distale.

## Patentansprüche

1. Gefäßtransplantat für die Aorta, das ein nächstgelegenes Ende und erste Mittel zum Verbinden an einer Distalöffnung beinhaltet, **dadurch gekennzeichnet, daß** das Gefäßtransplantat für die Aorta eine zwischen einem zu dem nächstgelegenen Ende und einem Distalteilstück nächstgelegenem Teilstück gelegene Verankerungsebene aufweist, die sich bis zu der Distalöffnung erstreckt, daß das nächstgelegene Teilstück gewellt ist, und daß das Distalteilstück zumindest teilweise nicht gewellt ist.

2. Gefäßtransplantat für die Aorta nach Anspruch 1, **dadurch gekennzeichnet, daß** das Distalteilstück des Gefäßtransplantats zumindest an der Stelle der ersten Verbindungsmittel, und bevorzugt über die gesamte Länge von der Verankerungsebene bis zur Distalöffnung nicht gewellt ist.

3. Gefäßtransplantat für die Aorta nach Anspruch 1 oder 2, **dadurch gekennzeichnet, daß** das Distalteilstück des Gefäßtransplantats mit zumindest einem Stent, und bevorzugt mit zumindest zwei Stents ausgestattet ist, die jeweilig nahe der Verankerungsebene und der Distalöffnung gelegen sind.

4. Gefäßtransplantat für die Aorta nach Anspruch 3, **dadurch gekennzeichnet, daß** das Distalteilstück des Gefäßtransplantats ein Distalende aufweist, das nicht gewellt ist, keine Stents aufweist und eine Länge von zumindest 20 mm umfaßt.

5. Gefäßtransplantat für die Aorta nach einem der vorangehenden Ansprüche 1 bis 4, **dadurch gekennzeichnet, daß** das Distalteilstück des Gefäßtransplantats für die Aorta zumindest an einem Großteil seiner Länge von Stents gehalten wird.

6. Gefäßtransplantat für die Aorta nach einem der vorangehenden Ansprüche 1 bis 5, **dadurch gekennzeichnet, daß** zumindest das nächstgelegene Teilstück des Gefäßtransplantats für die Aorta vorgebogen ist.

7. Gefäßtransplantat für die Aorta nach einem der vorangehenden Ansprüche 1 bis 6, **dadurch gekennzeichnet, daß** in dem nächstgelegenen Teilstück eine keilförmige Ebene der Transplantatseitenwand entfernt und die enthüllten gegenüberliegenden Kranzebenen zusammengeführt werden.

8. Gefäßtransplantat für die Aorta nach einem der vorangehenden Ansprüche 1 bis 7, **dadurch gekennzeichnet, daß** das nächstgelegene Teilstück eine Länge im Bereich von 10 bis 15 cm aufweist und daß das Distalteilstück, das die Distalendebene mit einer Länge im Bereich von 2 bis 3 cm beinhaltet, eine Länge im Bereich von 5 bis 10 cm umfaßt.

9. Gefäßtransplantat für die Aorta nach einem der vorangehenden Ansprüche 1 bis 8, **dadurch gekennzeichnet, daß** die Verankerungsebene eine das Gefäßtransplantat für die Aorta einkreisende, ringförmige Fläche aufweist.

10. Gefäßtransplantat für die Aorta nach einem der vorangehenden Ansprüche 1 bis 9, **dadurch gekennzeichnet, daß** die ringförmige Fläche nicht gewellt oder mit Stents versehen ist und eine Länge von zumindest 10 mm aufweist.

11. Gefäßtransplantat für die Aorta nach einem der Ansprüche 9 oder 10, **dadurch gekennzeichnet, daß** die Verankerungsebene eine Seitenfläche umfaßt, die sich von der ringförmigen Fläche in Richtung des nächstgelegenen Teilstücks erstreckt.

12. Gefäßtransplantat für die Aorta nach Anspruch 11, **dadurch gekennzeichnet, daß** ein aortisches Bogenteil sowie die Seitenfläche, des Gefäßtransplantats für die Aorta eine Markierung, wie etwa Radiopaque - Markierungen, oder eine vom Rest des Gefäßtransplantats verschiedene Oberflächenfarbe aufweist.

13. Gefäßtransplantat für die Aorta nach Anspruch 11, **dadurch gekennzeichnet, daß** die Seitenfläche eine Seitenöffnung beinhaltet.

14. Gefäßtransplantat für die Aorta nach einem der vorangehenden Ansprüche 1 bis 13, **dadurch gekennzeichnet, daß** in einem Montageabschnitt des Gefäßtransplantats für die Aorta das nächstgelegene Teilstück dem Distalteilstück angeglichen ist, um die ringförmige Verankerungsebene als eine Kranzebene darzustellen.

15. Gefäßtransplantat für die Aorta nach einem der vorangehenden Ansprüche 1 bis 14, **dadurch gekennzeichnet, daß** das Gefäßtransplantat eine nächstgelegene Verlängerung mit einer Herzklappe aufweist.

16. Gefäßtransplantat für die Aorta nach Anspruch 15, **dadurch gekennzeichnet, daß** das Gefäßtransplantat für die Aorta eine nächstgelegene Öffnung aufweist und daß die nächstgelegene Verlängerung mit der Herzklappe ein an der nächstgelegenen Öffnung separat an das Gefäßtransplantat anzubringendes Teil darstellt.

17. Gefäßtransplantat für die Aorta nach einem der vorangehenden Ansprüche, **dadurch gekennzeichnet, daß** das Gefäßtransplantat eine Distaltransplantatverlängerung mit zweiten Mitteln zum Verbinden aufweist, die bei Einrasten mit den ersten Mitteln zum Verbinden die nächstgelegene Transplantatverbindung mit dem Gefäßtransplantat für die Aorta verbindet.

18. Gefäßtransplantat für die Aorta nach Anspruch 17, **dadurch gekennzeichnet, daß** das Einrasten geometrisch ineinander greifende Teile der ersten und zweiten Mitteln zum Verbinden einbezieht, und zwar bevorzugt, so daß das Einrasten verbessert wird, wenn die Transplantatverlängerung in die Distalrichtung gezogen wird.
